# EUROPEAN PATENT APPLICATION

(11) **EP 2 727 614 A1**
(43) Date of publication of application: **07.05.2014**
(21) Application number: 12821755.1
(22) Date of filing: 08.08.2012
(51) Int. Cl.: A61M 1/34, A61M 1/14

(54) **BLOOD PURIFYING SYSTEM**

(30) Priority: 09.08.2011 JP 2011173806
(71) Applicant: Kawasumi Laboratories, Inc., Saiki-shi, Oita 876-0121 (JP)
(72) Inventor: TANIGUCHI, Kumiko, Kanagawa 2100855 (JP); TAKAHASHI, Masayuki, Kanagawa 2100855 (JP); TOKUMITSU, Satoshi, Kanagawa 2100855 (JP)
(74) Representative: Ahner, Philippe
(86) International application number: PCT/JP2012/070207
(87) International publication number: WO 2013/022024

(57) **Abstract**

It is an object of the present invention to provide a blood purifying apparatus capable of avoiding burdensome apparatus arrangement and reducing a burden on an operator while ensuring patient safety. The present invention provides a blood purifying apparatus (80) including a blood pump (81) for feeding blood on an arterial line toward a blood purifier, a dialysate pump (82) for feeding dialysate on a dialysate line toward a blood purifier, a waste fluid pump (83) for feeding out waste fluid in a waste fluid line, a first pump (84) for feeding a substitution fluid in a first line toward a venous line, a second pump (85) for feeding a replacement fluid in a second line toward the venous line, and a controller (90) configured to control driving of these pumps. The controller (90) controls the flow rate of the first pump (84) and the flow rate of the second pump (85) in an interlocking manner.

## Description

### TECHNICAL FIELD

The present invention relates to a blood purifying apparatus.

### BACKGROUND ART

Conventionally, as a blood purification therapy for purifying blood of a patient having acute renal failure and the like, a continuous hemodiafiltration (CHDF) therapy and the like have been conducted and many blood purifying apparatuses for use in such a blood purification therapy have been developed (see, e.g., Patent Documents 1 and 2).

Also, a new blood purification therapy called PDF (Plasma Dia-filtration) for purifying blood of a patient having acute hepatic failure and the like has been devised, and there are ongoing studies on this therapy (see, e.g., Non-Patent Document 1). The PDF uses a plasma separator having a hollow fiber membrane of a predetermined sieving coefficient, and dialysate and the like is perfused to an outer side of the hollow fiber membrane while at the same time performing a simple plasma exchange (PE).

### PRIOR ART DOCUMENTS

### PATENT DOCUMENTS

Patent Document 1: JP 2001-541 A
Patent Document 2: JP 2003-126247 A

### NON-PATENT DOCUMENTS

Non-Patent Document 1: Toyokazu Yoshioka et al. (13 others), "Multicenter study on effectiveness of Plasma Dia-Filtration (PDF)-Selective plasma filtration with dialysis", ICU & CCU Japanese Journal of Intensive Care Medicine, vol. 32 separate volume, 2008, p. 93-96

### SUMMARY OF THE INVENTION

### PROBLEMS TO BE SOLVED BY THE INVENTION

When actually performing the PDF, as shown in Fig. 1 of Non-Patent Document 1, it is necessary to provide a blood purifying apparatus having four pumps, namely a blood pump, a dialysate pump, a filtrate pump and a substitution fluid pump (hereinafter "conventional blood purifying apparatus") and to additionally provide a pump apparatus having an infusion pump for feeding a replacement fluid into a venous line. That is, it is necessary to provide two apparatuses every time. Thus, it is undeniable that an apparatus arrangement is burdensome.

Also, the respective pumps of the conventional blood purifying apparatus and the infusion pump for the replacement fluid are provided in separate apparatuses, respectively, and flow rates of the pumps are also separately controlled. Accordingly, in a case where the flow rate of the infusion pump is set in accordance with the flow rate of the pump (e.g., the blood pump) provided in the conventional blood purifying apparatus, when there is a change in the flow rate of the pump provided in the conventional blood purifying apparatus, the flow rate of the infusion pump needs to be adjusted manually. If an operator pays constant attention to the apparatus to manually adjust the flow rate of the infusion pump, there would be a substantial burden on the operator, as the PDF is performed for a relatively long time (e.g., 8 to 9 hours for one course). However, if the flow rate of the infusion pump is not adjusted, the blood homeostasis of the patient may be deteriorated, for example, a serum protein concentration may decrease, which may result in bringing detriment to the patient.

The present invention has been made to solve the above problems, and it is an object of the present invention is provide a blood purifying apparatus capable of avoiding a burdensome apparatus arrangement and reducing a burden on an operator while ensuring patient safety.

### MEANS FOR SOLVING THE PROBLEM

A blood purifying apparatus of the invention is for use in a blood purifying system including a blood purifier, an inside of which is partitioned into a blood flow passage and another flow passage, the blood purifier having a first inflow part communicating with one end of the blood flow passage, a first outflow part communicating with another end of the blood flow passage, a second inflow part communicating with one end of the other flow passage and a second outflow part communicating with another end of the other flow passage; an arterial line coupled to the first inflow part; a venous line coupled to the first outflow part; a dialysate line coupled to the second inflow part; a waste fluid line coupled to the second outflow part and through which waste fluid from the second outflow part passes; a first line coupled to the venous line and through which a first fluid passes; and a second line coupled to the arterial line, the venous line or the first line and through which a second fluid passes. The blood purifying apparatus includes a plurality of pumps and a controller configured to control driving of the pumps. The plurality of pumps includes a blood pump for feeding blood in the arterial line towards the blood purifier, a dialysate pump for feeding dialysate in the dialysate line towards the blood purifier, a waste fluid pump for feeding out the waste fluid in the waste fluid line, a first pump for feeding the first fluid in the first line towards the venous line, and a second pump for feeding the second fluid in the second line towards the arterial line, the venous line or the first line. The controller controls a flow rate of the first pump and a flow rate of the second pump in an interlocking manner.

Therefore, according to the blood purifying apparatus of the invention, five pumps described above are incorporated in the apparatus, and the first pump or the second pump can function as an infusion pump. Thus, it is not necessary to provide a pump apparatus having an infusion pump.

Also, according to the blood purifying apparatus of the invention, because the controller controls the flow rates of the first pump and the second pump in an interlocking manner, it is possible to automatically adjust the flow rate of the second pump in association with a change in the flow rate of the first pump (or to automatically adjust the flow rate of the first pump in association with a change in the flow rate of the second pump). That is, it is not necessary to stand by near the apparatus only for the purpose of adjusting the flow rate. Also, because the flow rates of the first pump and the second pump are correctly adjusted, it is possible to sufficiently ensure patient safety.

Therefore, the blood purifying apparatus of the invention is capable of avoiding a burdensome apparatus arrangement and reducing a burden on an operator while ensuring patient safety.

In the present description, the "first fluid" is a liquid that is different from the patient's blood, and the "second fluid" is a liquid that is different from the patient's blood and also different from the first fluid.

In the blood purifying apparatus of the invention, it is preferable that the controller further controls the flow rate of at least one of the first pump and the second pump and a flow rate of the waste fluid pump in an interlocking manner.

By this configuration, it is possible to automatically adjust the flow rates of the first pump and the second pump in accordance with a change in the flow rate of the waste fluid pump.

In the blood purifying apparatus of the invention, it is preferable that the controller further controls the flow rate of at least one of the first pump and the second pump and a flow rate of the blood pump in an interlocking manner.

By this configuration, it is possible to automatically adjust the flow rates of the first pump and the second pump in accordance with a change in the flow rate of the blood pump.

It is preferable that the blood purifying apparatus of the invention further includes a setting input portion for inputting and setting flow rates of the respective pumps, and that the controller adjusts the flow rates of the respective pumps, based on setting values input from the setting input portion.

By this configuration, for example, it is possible to change the flow rate of the second pump with the flow rate of the first pump being kept constant (i.e., to change a flow ratio of the first pump and the second pump) or to change a blood amount to be fed to the blood purifier with the flow ratios of all the pumps being kept the same, so that it is possible to provide a blood purifying apparatus capable of dealing with a wide range of user's intention.

### EFFECTS OF THE INVENTION

According to the blood purifying apparatus of the invention, because the first pump or the second pump can function as an infusion pump, it is not necessary to provide a pump apparatus having an infusion pump. Also, since the controller controls the flow rates of the first pump and the second pump in an interlocking manner, it is possible to automatically adjust the flow rate of the second pump in association with a change in the flow rate of the first pump (or to automatically adjust the flow rate of the first pump in association with a change in the flow rate of the second pump). As a result, it is possible to avoid a burdensome apparatus arrangement and reducing a burden on an operator while ensuring patient safety.

### BRIEF DESCRIPTION OF THE DRAWINGS

Fig. 1 is a diagram illustrating a configuration of a blood purifying system 1 including a blood purifying apparatus 80 according to an embodiment of the present invention.
Figs. 2(a) and 2(b) illustrate a blood purifier 10, in which Fig. 2(a) is a side view of the blood purifier 10 and Fig. 2(b) is a diagram illustrating an internal structure of the blood purifier 10.
Fig. 3 is a block diagram illustrating an electrical configuration of the blood purifying apparatus 80.

### EMBODIMENTS OF THE INVENTION

Hereinafter, a blood purifying apparatus of the invention will be described with reference to an embodiment shown in the drawings.

### [Embodiment]

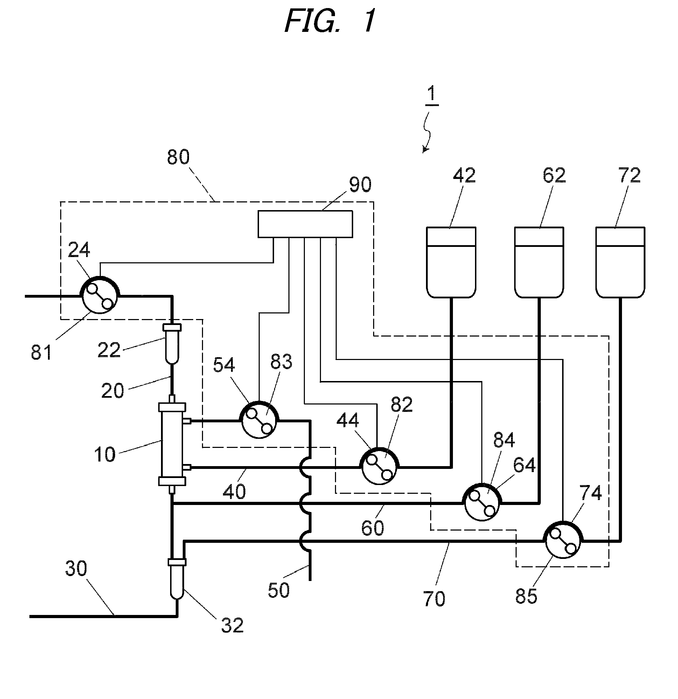

First, a configuration of a blood purifying system 1 for purifying blood will be described with reference to Figs. 1 to 3.

Fig. 1 is a diagram illustrating a configuration of a blood purifying system 1 including a blood purifying apparatus 80 according to an embodiment of the present invention.

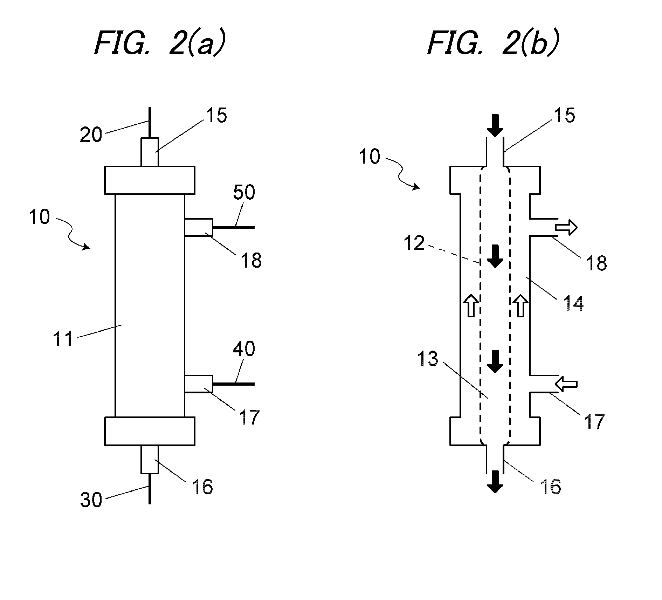

Figs. 2(a) and 2(b) illustrate a blood purifier 10. Fig. 2(a) is a side view of the blood purifier 10 and Fig. 2(b) is a diagram illustrating an internal structure of the blood purifier 10. In Fig. 2(b), in order to easily understand the invention, only one selective separation membrane 12 of a plurality of selective separation membranes 12 arranged in the blood purifier 10 is illustrated.

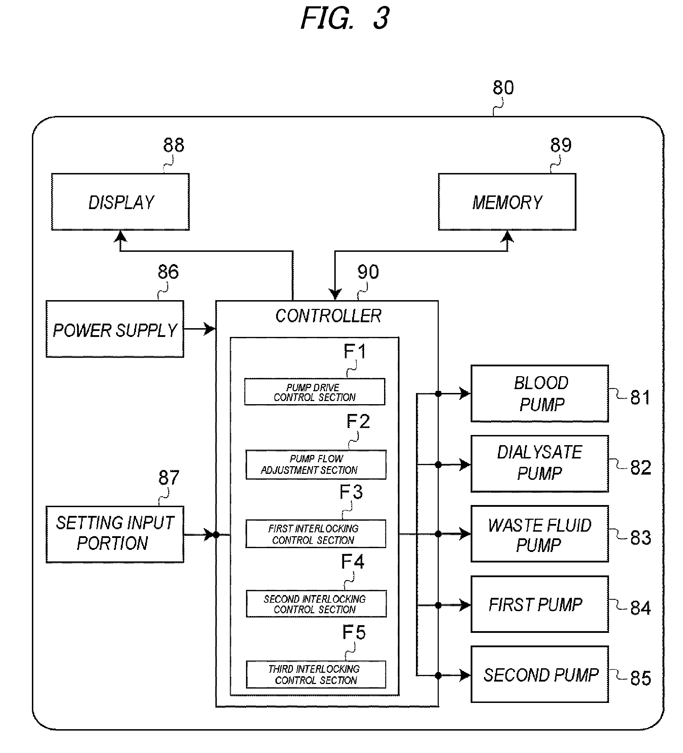

Fig. 3 is a block diagram illustrating an electrical configuration of the blood purifying apparatus 80.

As shown in Figs. 1, 2(a) and 2(b), the blood purifying system 1 includes the blood purifier 10, an arterial line 20 coupled to a first inflow part 15 of the blood purifier 10, a venous line 30 coupled to a first outflow part 16 of the blood purifier 10, a dialysate line 40 coupled to a second inflow part 17 of the blood purifier 10, a dialysate reservoir 42 capable of reserving dialysate, a waste fluid line 50 coupled to a second outflow part 18 of the blood purifier 10 and through which waste fluid from the second outflow part 18 passes, a first line 60 coupled to the venous line 30 and through which a substitution fluid passes as a first fluid, a substitution fluid reservoir 62 capable of reserving the substitution fluid, a second line 70 coupled to the venous line 30 and through which a replacement fluid passes as a second fluid, a replacement fluid reservoir 72 capable of reserving the replacement fluid and a blood purifying apparatus 80.

As shown in Figs. 2(a) and 2(b), the blood purifier 10 includes a cylindrical housing 11, the selective separation membrane 12 arranged inside the housing 11, the first inflow part 15 provided at one end portion of the housing 11, the first outflow part 16 provided at the other end portion of the housing 11, the second inflow part 17 provided on a side surface of the housing 11 at a position near the first outflow part 16, and the second outflow part 18 provided on the side surface of the housing 11 at a position near the first inflow part 15.

The selective separation membrane 12 is, for example, a hollow fiber membrane. Although illustration in the drawings is omitted, a plurality of selective separation membranes 12 is arranged inside the housing 11 to extend along a longitudinal direction of the housing 11.

As shown in Fig. 2(b), the inside of the blood purifier 10 is partitioned into a blood flow passage 13 and another flow passage 14 by the selective separation membrane 12. That is, the inner side of the selective separation membrane 12 is the blood flow passage 13 and the outer side of the selective separation membrane 12 is the other flow passage 14. The first inflow part 15 and the first outflow part 16 communicate with the blood flow passage 13, and the second inflow part 17 and the second outflow part 18 communicate with the other flow passage 14. The blood purifier 10 is configured such that, at the time of blood purification, a flowing direction (a black arrow direction in Fig. 2(b)) of the blood flow passage 13 and a flowing direction (a white arrow direction in Fig. 2(b)) of the other flow passage 14 are opposite to one another (i.e., opposed flows are formed).

As the blood purifier 10, for example, Evacure EC-2A (an albumin sieving coefficient is about 0.3, "Evacure" is a registered trademark of Kawasumi Laboratories, Inc.) may be suitably used. It is preferable to use a blood purifier including a selective separation membrane having albumin sieving coefficient of larger than 0 and smaller than 1.

As shown in Fig. 1, an air trap 22 and a rolling tube portion 24 are provided in the arterial line 20. Although not shown in Fig. 1, in the arterial line 20, an anticoagulant injection line for introducing anticoagulant into the line 20 is coupled, and a pressure detector for detecting a pressure in the arterial line 20 is provided.

An air trap 32 is provided in the venous line 30. Although not shown in Fig. 1, a bubble detector is provided in the venous line 30, and a venous pressure sensor is coupled to the air trap 32.

Rolling tube portions 44, 54 are provided in the dialysate line 40 and the waste fluid line 50, respectively. Although not shown in Fig. 1, in the waste fluid line 50, a pressure detector for detecting a pressure in the waste fluid line 50 is provided.

Rolling tube portions 64, 74 are provided in the first line 60 and the second line 70, respectively. An end portion of the first line 60 is coupled to the venous line 30 at a position between a part where the blood purifier 10 is connected and the air trap 32. An end portion of the second line 70 is connected to the air trap 32 of the venous line 30.

The dialysate reservoir 42, the substitution fluid reservoir 62 and the replacement fluid reservoir 72 are detachably and exchangeably attached to end portions of the dialysate line 40, the first line 60 and the second line 70, respectively. The dialysate reservoir 42, the substitution fluid reservoir 62 and the replacement fluid reservoir 72 are, for example, plastic reservoir bags. As the dialysate and the substitution fluid, for example, Sublood-BS ("Sublood" is a registered trademark of Fuso Pharmaceutical Industries, Ltd.) may be suitably used. As the replacement fluid, for example, fresh frozen plasma (FFP), or albumin solutions of 5%, 20% and 25% and the like may be suitably used. With regard to the dialysate, the substitution fluid and the replacement fluid, those other than the examples illustrated herein may of course be used also.

As shown in Figs. 1 and 3, the blood purifying apparatus 80 includes a blood pump 81 for feeding blood in the arterial line 20 towards the blood purifier 10, a dialysate pump 82 for feeding dialysate in the dialysate line 40 towards the blood purifier 10, a waste fluid pump 83 for feeding out waste fluid in the waste fluid line 50, a first pump 84 for feeding a substitution fluid in the first line 60 towards the venous line 30, a second pump 85 for feeding a replacement fluid in the second line 70 towards the venous line 30, a power supply 86 for feeding power to each part in the blood purifying apparatus 80, a setting input portion 87 arranged on an outer surface of the housing of the blood purifying apparatus 80 and configured to receive an input from an operator, a display 88 arranged on the outer surface of the housing of the blood purifying apparatus 80 and configured to display various information such as temperature information on the blood flowing in the line and venous pressure information measured by the venous pressure sensor, a memory 89 that stores performance data and the like of the blood purifier being used, and a controller 90 that collectively controls the respective parts in the blood purifying apparatus 80.

In Fig. 1, the lines extending from the controller 90 to the respective pumps 81 to 85 indicate signal lines.

The blood pump 81, the dialysate pump 82, the waste fluid pump 83, the first pump 84 and the second pump 85 are so-called roller pumps. Although illustration in the drawings is omitted, a roller part of the blood pump 81 rotates while contacting the rolling tube portion 24 of the arterial line 20 (compresses the rolling tube portion 24), thereby feeding the fluid (blood) in the arterial line 20 in a predetermined direction. Functions of the other pumps 82 to 85 are similar to that of the blood pump 81.

Although illustration in the drawings is omitted, the setting input portion 87 is configured such that the flow rates of the respective pumps, a flow rate interlocking ratio among the pumps, and other setting values can be input. The setting input portion 87 and the display 88 may be configured separately, or may be a touch panel type display in which an input part and a display part are integrated.

As shown in Fig. 3, the controller 90 has at least a pump drive control section F1 configured to control driving of the five pumps 81 to 85, a pump flow adjustment section F2 configured to adjust the flow rates of the respective pumps based on setting values input from the setting input portion 87, a first interlocking control section F3 configured to control the flow rates of the first pump 84 and the second pump 85 interlockingly with the flow rate of the blood pump 81, a second interlocking control section F4 configured to control the flow rates of the first pump 84 and the second pump 85 interlockingly with the flow rate of the waste fluid pump 83, and a third interlocking control section F5 configured to control the flow rates of the first pump 84 and the second pump 85 in an interlocking manner.

The pump drive control section F1 reads out the flow rate information of the five pumps 81 to 85 stored in the memory 89, and controls the driving of the respective pumps 81 to 85 based on the information. That is, based on the set flow rates of the respective pumps 81 to 85 prestored in the memory 89, the pump drive control section controls the rotation drives of the respective pumps 81 to 85.

The setting input portion 87 generates setting value information associated with the setting values that are input. The pump flow adjustment section F2 acquires the setting value information generated by the setting input portion 87, and adjusts the flow rates of the respective pumps 81 to 85 based on the setting value information.

The first interlocking control section F3 controls the flow rate of at least one of the first pump 84 and the second pump 85 in accordance with the adjustment of the flow rate of the blood pump 81.

For example, when a negative pressure is generated by a kink of a tube and like and a variation in pressure of the arterial line 20 is detected by the pressure detector provided in the arterial line 20, the detection information is provided to the pump drive control section F1. The pump drive control section F1 reads out the flow rate information stored in the memory 89 and controls the drive of the blood pump 81 so that the blood pump 81 is rotated at a safer flow rate, based on the read information. In order to avoid the unbalance between an amount of blood to be removed and an amount of fluid to be replenished or replaced, which may be caused by adjusting the flow rate of the blood pump 81, the first interlocking control section F3 controls the flow rate of at least one of the first pump 84 and the second pump 85 in an interlocking manner.

Also, when the flow rate of the blood pump 81 is adjusted by the pump flow adjustment section F2, the first interlocking control section F3 can control the flow rate of at least one of the first pump 84 and the second pump 85 in an interlocking manner. That is, simply by setting the flow rate of the blood pump 81 by the setting input portion 87, the flow rates of the first pump 84 and the second pump 85 are also changed. Cases where the flow rate of the blood pump 81 is adjusted by the pump flow adjustment section F2 include, for example, a case where it is desirable to change an amount of blood to be removed in view of a condition of the patient.

The second interlocking control section F4 controls the flow rate of at least one of the first pump 84 and the second pump 85 in accordance with the adjustment of the flow rate of the waste fluid pump 83.

For example, when a clogging occurs in the blood purifier and a variation in pressure of the waste fluid line 50 is detected by the pressure detector provided in the waste fluid line 50, the detection information is provided to the pump drive control section F1. The pump drive control section F1 reads out the flow rate information stored in the memory 89 and controls the drive of the waste fluid pump 83 so that the waste fluid pump 83 is rotated at a suitable flow rate based on the read information. In order to avoid the unbalance between an amount of waste fluid and an amount of fluid to be substituted or replaced, which may be caused by adjusting the flow rate of the waste fluid pump 83, the second interlocking control section F4 controls the flow rates the first pump 84 and the second pump 85 in an interlocking manner.

Also, when the flow rate of the waste fluid pump 83 is adjusted by the pump flow adjustment section F2, the second interlocking control section F4 can control the flow rate of at least one of the first pump 84 and the second pump 85 in an interlocking manner. That is, simply by setting the flow rate of the waste fluid pump 83 by the setting input portion 87, the flow rates of the first pump 84 and the second pump 85 are also changed. Cases where the flow rate of the waste fluid pump 83 is adjusted by the pump flow adjustment section F2 include, for example, a case where it is desirable to change an amount of water to be removed due to a condition change, such as a blood pressure drop of the patient.

The third interlocking control section F5 controls, in accordance with the adjustment of the flow rate of one of the first pump 84 and the second pump 85 by the pump flow adjustment section F2, the flow rate of the other pump.

According to the blood purifying apparatus 80 of this embodiment configured as described above, the apparatus is provided with the five pumps 81 to 85, and the second pump 85 can function as an infusion pump. Therefore, it is not necessary to provide a pump apparatus having an infusion pump.

Also, according to the blood purifying apparatus 80 of this embodiment, since the controller 90 has the third interlocking control section F5 configured to control the flow rates of the first pump 84 and the second pump 85 in an interlocking manner, it is possible to automatically adjust the flow rate of the second pump 85 in association with a change in the flow rate of the first pump 84 (or to automatically adjust the flow rate of the first pump 84 in association with a change in the flow rate of the second pump 85). That is, it is not necessary to stand by near the apparatus only for the purpose of adjusting the flow rate. Also, because the flow rates of the first pump 84 and the second pump 85 are correctly adjusted, it is possible to sufficiently ensure patient safety.

Therefore, the blood purifying apparatus 80 of the embodiment is a blood purifying apparatus capable of avoiding a burdensome apparatus arrangement and reducing a burden on an operator while ensuring patient safety.

In the blood purifying apparatus 80 of the embodiment, the controller 90 further has the first and second interlocking control sections F3, F4. Hence, it is possible to automatically adjust the flow rates of the first pump 84 and the second pump 85 in accordance with a change in the flow rate of the blood pump 81 or the waste fluid pump 83.

In the blood purifying apparatus 80 of the embodiment, the controller 90 further has the pump flow adjustment section F2. Thus, for example, it is possible to change the flow rate of the second pump 85 with the flow rate of the first pump 84 being kept constant (i.e., to change a flow ratio of the first pump 84 and the second pump 85) or to change the blood amount to be fed to the blood purifier 10 with the flow ratios of all the pumps 81 to 85 being kept the same, so that it is possible to provide a blood purifying apparatus capable of dealing with a wide range of user's intention.

Here, features of the embodiment of the blood purifying apparatus according to the present invention are summarized and listed in the following sections i to iv.
[i] The blood purifying apparatus 80 for use in the blood purifying system 1 including the blood purifier 10, the inside of which is partitioned into the blood flow passage 13 and the other flow passage 14, the blood purifier 10 having the first inflow part 15 communicating with one end of the blood flow passage 13, the first outflow part 16 communicating with the other end of the blood flow passage 13, the second inflow part 17 communicating with one end of the other flow passage 14, and the second outflow part 18 communicating with the other end of the other flow passage 14; the arterial line 20 coupled to the first inflow part 15; the venous line 30 coupled to the first outflow part 16; the dialysate line 40 coupled to the second inflow part 17; the waste fluid line 50 coupled to the second outflow part 18 and through which waste fluid from the second outflow part 18 passes; the first line 60 coupled to the venous line 30 and through which the first fluid passes, and the second line 70 coupled to the arterial line 20, the venous line 30 or the first line 50 and through which the second fluid passes, the blood purifying apparatus including a plurality of pumps and the controller 90 configured to control driving of the pumps, wherein the plurality of pumps includes the blood pump 81 for feeding blood in the arterial line 20 towards the blood purifier 10, the dialysate pump 82 for feeding dialysate in the dialysate line 40 towards the blood purifier 10, the waste fluid pump 83 for feeding out the waste fluid in the waste fluid line 50, the first pump 84 for feeding the first fluid in the first line 60 towards the venous line 30, and the second pump 85 for feeding the second fluid in the second line 70 towards the arterial line 20, the venous line 30 or the first line 60, and wherein the controller 90 controls a flow rate of the first pump 84 and a flow rate of the second pump 95 in an interlocking manner.
[ii] The blood purifying apparatus 80 according to [i] described above, in which the controller 90 further controls the flow rate of at least one of the first pump 84 and the second pump 85 and a flow rate of the waste fluid pump 83 in an interlocking manner.
[iii] The blood purifying apparatus 80 of according to [i] or [ii] described above, in which the controller 90 further controls the flow rate of at least one of the first pump 84 and the second pump 85 and a flow rate of the blood pump 81 in an interlocking manner.
[iv] The blood purifying apparatus 80 according to any one of [i] to [iii] described above, further including a setting input portion 87 for inputting and setting flow rates of the respective pumps, in which the controller 90 adjusts the flow rates of the respective pumps 81 to 85, based on setting values input from the setting input portion 87.

While a blood purifying apparatus of the present invention has been described with reference to the above embodiment, the present invention is not limited to the above embodiment, and may be implemented in various embodiments without departing from the gist of the invention. For example, following modifications can be made.
(1) In the above embodiment, the second line 70 is connected to the venous line 30 (the air tap 32). However, the present invention is not limited thereto. The second line may be connected to any position between the end portion of the first line 60 (the part where the venous line 30 is connected) and the rolling tube portion 64 (the position at which the first pump 84 is arranged) or may be connected to any position in the arterial line 20.
(2) In the above embodiment, the blood purifier having the selective separation membrane consisting of the hollow fiber membrane has been described as an example of the blood purifier. However, the present invention is not limited thereto. For example, the invention can be also applied to a blood purifier having a selective separation membrane other than the hollow fiber membrane, such as a flat membrane (having a plurality of layers of flat plate-type membranes) and a tubular membrane.
(3) In the above embodiment, the dialysate reservoir 42 and the substitution fluid reservoir 62 have been described as being separately provided as an example. However, the present invention is not limited thereto. For example, when contents of the dialysate reservoir and the substitution fluid reservoir are the same, they may be integrated.
(4) In the above embodiment, the controller 90 having the pump drive control section F1, the pump flow adjustment section F2, and the first to third interlocking control sections F3 to F5 has been described as an example. However, the present invention is not limited thereto. In addition, for example, the controller may further have a fourth interlocking control section configured to control the flow rates of the first pump 84 and the second pump 85 interlockingly with the flow rate of the dialysate pump 82. In this case, it is possible to automatically adjust the flow rates of the first pump and the second pump in accordance with a change in the flow rate of the dialysate pump.
(5) In the above embodiment, the so-called roller pumps are described as being used as the respective pumps 81 to 85 as an example. However, the present invention is not limited thereto. For example, the other well-known fluid feeding means such as a syringe pump may also be used.
(6) In the above embodiment, a reservoir condition detector (for example, a weight scale, an ultrasonic sensor and the like) that detects the lowering of the fluid amount in the reservoir may be further provided in the blood purifying apparatus, and the driving of the pump for the reservoir in which the fluid amount is lowered may be slowed down, based on the detection information of the reservoir condition detector. In this case, for example, when it is detected that the fluid amount of the substitution fluid reservoir 62 or replacement fluid reservoir 72 is reduced, it is possible to slow down the drive of the pump (one of the pumps) for the reservoir in which the fluid amount is lowered, based on the detection information, and also to slow down the drive of the other pump. In this way, even when the fluid amount of the reservoir is reduced, it is possible to prevent the reservoir from being empty.

The reservoir condition detector may be arranged in the blood purifying system, separately from the blood purifying apparatus.
(7) In the above embodiment, a pressure detector that detects a pressure at a predetermined position in the line and a transmembrane pressure calculation unit that calculates a transmembrane pressure (TMP) of the blood purifier on the basis of the measuring information from the pressure detector may be provided in the blood purifying apparatus, and when the calculated transmembrane pressure exceeds a predetermined value, the drives of the first pump 84 and the second pump 85 may be slowed down. When the transmembrane pressure exceeds a predetermined value, there is a possibility that the clogging due to the protein and the like occurs in the selective separation membrane of the blood purifier. Hence, for example, if one of the substitution fluid reservoir 62 and the replacement fluid reservoir 72 is precharged with a fluid having a clogging removing effect, when the transmembrane pressure exceeds a predetermined value, the fluid having a clogging removing effect can be fed to the line, so that it is possible to solve the clogging of the selective separation membrane.

The pressure detector and the transmembrane pressure calculation unit may be arranged in the blood purifying system, separately from the blood purifying apparatus.
(8) In the above embodiment, a lapse time measuring unit that measures time elapsed from starting of the fluid feed from the reservoir to the present time and a fluid feed time setting unit that sets a time period (a fluid feed time period) from the starting of the fluid feed to the stopping of the fluid feed may be provided in the blood purifying apparatus, and when the measured elapsed time exceeds the fluid feed time period, the flow rates of the first pump 84 and the second pump 85 may be reduced or stopped. Alternatively, an actual amount measuring unit that measures a fluid feed amount (an actual amount) from the starting of the fluid feed from the reservoir to the present time and a reference amount setting unit that sets a fluid feed amount (a reference amount) from the starting of the fluid feed to the stopping of the fluid feed may be provided in the blood purifying apparatus, and when the measured actual amount exceeds the reference amount, the flow rates of the first pump 84 and the second pump 85 may be reduced or stopped. In this case, it is possible to adjust the fluid feed amount from the substitution fluid reservoir 62 or the replacement fluid reservoir 72, depending on the elapsed time or fluid feed amount.

The lapse time measuring unit and the fluid feed time setting unit or the actual amount measuring unit and the reference amount setting unit may be arranged in the blood purifying system, separately from the blood purifying apparatus.

While the present invention has been described in detail with reference to the specific embodiments, it is apparent to those skilled in the art that various changes and modifications can be made without departing from the spirit and scope of the invention. The application is based on a Japanese Patent Application No. 2011-173806 filed on August 9, 2011, the content of which is incorporated herein by reference.

### EXPLANATION OF REFERENCE SIGNS

- 1:: blood purifying system
- 10:: blood purifier
- 11:: housing
- 12:: selective separation membrane
- 13:: blood flow passage
- 14:: other flow passage
- 15:: first inflow part
- 16:: first outflow part
- 17:: second inflow part
- 18:: second outflow part
- 20:: arterial line
- 22, 32:: air trap
- 24, 44, 54, 64, 74:: rolling tube portion
- 30:: venous line
- 40:: dialysate line
- 42:: dialysate reservoir
- 50:: waste fluid line
- 60:: first line
- 62:: substitution fluid reservoir
- 70:: second line
- 72:: replacement fluid reservoir
- 80:: blood purifying apparatus
- 81:: blood pump
- 82:: dialysate pump
- 83:: waste fluid pump
- 84:: first pump
- 85:: second pump
- 86:: power supply
- 87:: setting input portion
- 88:: display
- 89:: memory
- 90:: controller
- F1:: pump drive control section
- F2:: pump flow adjustment section
- F3:: first interlocking control section
- F4:: second interlocking control section
- F5:: third interlocking control section

## Claims

1. A blood purifying apparatus for use in a blood purifying system, the blood purifying system comprising:
a blood purifier, an inside of which is partitioned into a blood flow passage and another flow passage, the blood purifier having a first inflow part communicating with one end of the blood flow passage, a first outflow part communicating with another end of the blood flow passage, a second inflow part communicating with one end of the other flow passage and a second outflow part communicating with another end of the other flow passage;
an arterial line coupled to the first inflow part;
a venous line coupled to the first outflow part;
a dialysate line coupled to the second inflow part;
a waste fluid line coupled to the second outflow part and through which waste fluid from the second outflow part passes;
a first line coupled to the venous line and through which a first fluid passes; and
a second line coupled to the arterial line, the venous line or the first line and through which a second fluid passes,
the blood purifying apparatus comprising a plurality of pumps and a controller configured to control driving of the pumps,
wherein the plurality of pumps comprises:
a blood pump for feeding blood in the arterial line towards the blood purifier,
a dialysate pump for feeding dialysate in the dialysate line towards the blood purifier,
a waste fluid pump for feeding out the waste fluid in the waste fluid line,
a first pump for feeding the first fluid in the first line towards the venous line, and
a second pump for feeding the second fluid in the second line towards the arterial line, the venous line or the first line, and
wherein the controller controls a flow rate of the first pump and a flow rate of the second pump in an interlocking manner.

2. The blood purifying apparatus according to claim 1, wherein the controller further controls the flow rate of at least one of the first pump and the second pump and a flow rate of the waste fluid pump in an interlocking manner.

3. The blood purifying apparatus according to claim 1 or 2, wherein the controller further controls the flow rate of at least one of the first pump and the second pump and a flow rate of the blood pump in an interlocking manner.

4. The blood purifying apparatus according to any one of claims 1 to 3, further comprising a setting input portion for inputting and setting flow rates of the respective pumps,
wherein the controller adjusts the flow rates of the respective pumps, based on setting values input from the setting input portion.
